(19) Europäisches Patentamt
European Patent Office
Office européen des brevets

(11) Veröffentlichungsnummer: **0 041 652**
**B1**

(12) **EUROPÄISCHE PATENTSCHRIFT**

(45) Veröffentlichungstag der Patentschrift: **22.02.84**

(21) Anmeldenummer: **81104077.3**

(22) Anmeldetag: **27.05.81**

(51) Int. Cl.³: **C 07 D 409/04,** C 07 D 405/02, C 07 D 233/58, C 07 D 233/64, A 61 K 31/415 // (C07D409/04, 233/64, 333/22),(C07D405/02, 233/64, 317/58),(C07D405/02, 233/64, 319/18)

(54) **Neue Imidazolderivate, ihre Herstellung und diese Derivate enthaltende Arzneimittel.**

(30) Priorität: **05.06.80 CH 4347/80**

(43) Veröffentlichungstag der Anmeldung: **16.12.81 Patentblatt 81/50**

(45) Bekanntmachung des Hinweises auf die Patenterteilung: **22.02.84 Patentblatt 84/8**

(84) Benannte Vertragsstaaten: **AT BE CH DE FR GB IT LI LU NL SE**

(56) Entgegenhaltungen: **DE - A - 2 733 466**

(73) Patentinhaber: **F. HOFFMANN-LA ROCHE & CO. Aktiengesellschaft, CH-4002 Basel (CH)**

(72) Erfinder: **Derungs, Romano, Dr., Bäumliweg 18, CH-4125 Riehen (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 041 652 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Neue Imidazolderivate, ihre Herstellung und diese Derivate enthaltende Arzneimittel

Die vorliegende Erfindung betrifft neue Imidazolderivate der Formel

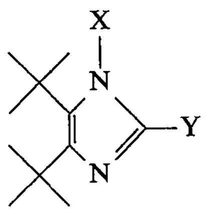

(I)

worin X Wasserstoff oder $C_{1-4}$-n-Alkyl und Y gegebenenfalls durch Methyl oder Fluor substituiertes Thienyl oder eine Gruppe der Formel $Y^1$

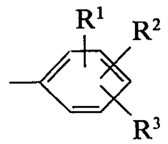

($Y^1$)

ist, worin $R^1$, $R^2$ und $R^3$ Wasserstoff, Methyl, Fluor, Hydroxy, Methoxy oder zwei benachbarte Reste $R^1$, $R^2$ oder $R^3$ zusammen Methylendioxy oder Äthylendioxy sind oder einer der Reste $R^1$, $R^2$ und $R^3$ Mono oder Di-($C_{1-4}$-n-alkyl)-amino ist und die zwei anderen Wasserstoff sind,
und physiologisch verträgliche Säureadditionssalze davon.

Der hier verwendete Ausdruck $C_{1-4}$-n-Alkyl bezieht sich auf die geradkettigen Alkylreste Methyl, Äthyl, Propyl und Butyl.

Unter den Verbindungen der Formel I sind diejenigen bevorzugt, worin X Wasserstoff und solche, worin Y eine Gruppe der Formel $Y^1$, insbesondere p-Fluorphenyl, ist.

Besonders bevorzugt ist Di-t-butyl-2-(4-fluorphenyl)-imidazol.

Die Erfindung betrifft weiterhin ein Verfahren zur Herstellung der genannten Verbindungen sowie pharmazeutische Präparate auf der Basis der genannten Verbindungen.

Beispiele physiologisch verträglicher Säureadditionssalze sind Mineralsäuresalze, wie Hydrochloride, Hydrobromide, Sulfate und Phosphate, Salze organischer Sulfonsäuren, wie Alkyl- oder Arylsulfonate, und Carbonsäuresalze, wie Succinate oder Citrate.

Die genannten Verbindungen können erfindungsgemäß dadurch hergestellt werden, daß man ein Thiepinoimidazol der Formel

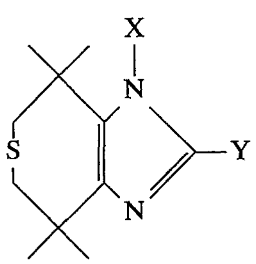

(II)

worin X und Y die obige Bedeutung haben,
oder ein Säureadditionssalz davon mit einem Entschwefelungskatalysator behandelt und gewünschtenfalls eine Verbindung der Formel I in ein Säureadditionssalz überführt.

Beispiele von Entschwefelungskatalysatoren sind Metallkatalysatoren wie Nickel- oder Palladium-Katalysatoren, vorzugsweise Nickel-Katalysatoren, insbesondere Raney-Nickel. Die Entschwefelung wird zweckmäßig in einem Lösungsmittel, vorzugsweise einem polaren Lösungsmittel, wie Dioxan oder Tetrahydrofuran, bei einer Temperatur bis zu Rückflußtemperatur durchgeführt.

Die Verbindungen der Formel II, worin X Wasserstoff ist, können durch Umsetzung von 3,3,6,6-Tetramethyl-4,5-thiepandion mit dem Aldehyd $Y-CH=O$ in Gegenwart von Ammoniumionen, vorzugsweise in Gegenwart eines Ammoniumsalzes, wie Ammoniumacetat, in einem polaren Lösungsmittel, wie Dimethylsulfoxyd oder Dimethylformamid, bei einer Temperatur bis zu Rückflußtemperatur hergestellt werden. Die erhaltenen Verbindungen der Formel II können durch Umsetzung mit einem Alkalimetallhydrid, wie NaH, in einem Lösungsmittel, wie Dimethylformamid, und Umsetzung der erhaltenen Verbindung mit einem Alkylhalogenid, wie Methyljodid, N-alkyliert werden.

Die Verbindungen der Formel I und die physiologisch verträglichen Salze davon können als

Heilmittel Verwendung finden. Sie hemmen die Aggregation der Blutplättchen und können daher zur Verhütung von Thrombosen verwendet werden.

Die Verbindungen der Formel I können als Heilmittel z. B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z. B. Wasser, Gelatine, Gummi arabicum, Milchzucker, Stärke, Magnesiumstearat, Talk, pflanzliche Öle, Polyalkylenglykole oder Vaseline enthalten. Die pharmazeutischen Präparate können in fester Form, z. B. als Tabletten, Dragées, Suppositorien, Kapseln; oder in flüssiger Form, z. B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten. Die orale Verabreichung der erfindungsgemäßen Verbindungen ist bevorzugt. Für den Erwachsenen kommen eine orale Tagesdosis von 0,5 bis 30 mg/kg und eine parenterale Tagesdosis von 0,05 bis 10 mg/kg in Frage.

Die aggregationshemmende Wirkung wurde nach der Aggregometer-Methode von BORN [Nature 194, 927 (1962)] und MICHAL und BORN [Nature 231, 220 (1971)] nachgewiesen. Die maximale Aggregationsgeschwindigkeit wurde als Versuchsparameter genommen und die effektive Konzentration ($EC_{50}$) aus Dosis-Wirkungskurven ermittelt.

Menschliches plättchenreiches Plasma wurde aus citriertem venösem Blut durch Zentrifugieren erhalten. Die Versuche wurden mit Suspensionen der Testsubstanzen in 0,9% NaCl durchgeführt. 0,18 ml Citratplasma wurden mit 10 µl Suspension der Testverbindungen versetzt und 10 Minuten bei 37° C inkubiert, worauf die Aggregation durch Zusatz von 10 µl einer Suspension von Kollagen-Fibrillen ausgelöst wurde.

Die Resultate sind in der nachstehenden Tabelle wiedergegeben.

Tabelle

Kollagen-induzierte Blutplättchenaggregation

| Verbindung | $EC_{50}$ (µM) |
|---|---|
| 4,5-Di-t-butyl-2-phenylimidazol | 0,6 |
| 4,5-Di-t-butyl-2-(4-fluorphenyl)-imidazol | 0,5 |
| 4,5-Di-t-butyl-2-(3,4-methylendioxy-phenyl)-imidazol | 0,55 |
| 4,5-Di-t-butyl-2-(5-methoxy-3,4-methylendioxyphenyl)-imidazol | 14,4 |
| 4,5-Di-t-butyl-2-(3,4,5-trimethoxy-phenyl)-imidazol | 0,72 |

Beispiel 1

5 g 2-Phenyl-4,5,7,8-tetrahydro-4,4,8,8-tetramethyl-1-H-thiepino[4,5-d]imidazol und 200 ml Dioxan werden unter Rühren in Gegenwart von Nickel-Katalysator (erhalten durch Entwässerung von 85 g feuchtem Nickel-Katalysator mit 300 ml Dioxan, Aufwirbeln gefolgt von Sedimentation des Katalysators und Absaugen des Lösungsmittels) 20 Stunden bei Rückflußtemperatur erhitzt.

Man läßt dann den Katalysator in der Reaktionslösung sedimentieren. Die Lösung wird abgesaugt. Der Katalysator wird mit 200 ml Dioxan versetzt und unter Rühren zum Sieden erhitzt. Nach dem Absetzen des Katalysators wird die Lösung wieder abgesaugt. Diese Prozedur wird nochmals durchgeführt.

Die vereinigten Extrakte werden filtriert und eingeengt. Nach Umkristallisation aus n-Heptan erhält man 1,2 g 4,5-Di-t-butyl-2-phenylimidazol, Smp. 156—158° C.

Zur Herstellung des Hydrochlorids werden 604 mg der Base in Äther gelöst und die Lösung durch Zutropfen ätherischer Salzsäure neutralisiert. Der Niederschlag wird abfiltriert und mit Äther und Äthanol aufgeschwemmt. Man erhält 680 mg des Hydrochlorids, Smp. 250—255° C (Zersetzung).

Das Ausgangsthiepinoimidazol kann wie folgt hergestellt werden:

Man löst 16 g 3,3,6,6-Tetramethyl-4,5-thiepandion und 8 g Benzaldehyd in 200 ml Dimethylsulfoxyd, fügt unter Rühren 60 g wasserfreies Ammoniumacetat zu und erhitzt auf 90°. Das Reaktionsgemisch wird nach dem Abkühlen unter Rühren in Eiswasser gegossen, die Lösung mit konzentrierter

Natronlauge alkalisch gestellt und mit Äther extrahiert. Die organische Phase wird mit Eiswasser gewaschen und zur Trockene eingeengt. Der Rückstand wird mit Petroläther überschichtet und mit einem Glasstab gerieben. Der abfiltrierte Niederschlag wird aus Toluol umkristallisiert. Man erhält 2-Phenyl-4,5,7,8-tetrahydro-4,4,8,8-tetramethyl-1-H-thiepino[4,5-d]imidazol, Smp. 225 – 227° C. 680 mg des Hydrochlorids, Smp. 250 – 255° C (Zersetzung).

Beispiel 2

In zu Beispiel 1 analoger Weise erhält man:

4,5-Di-t-butyl-2-(4-fluorphenyl)-imidazol, Smp. 185° C;
Smp. des Hydrochlorids 240° C (Zersetzung),
4,5-Di-t-butyl-2-(4-methoxyphenyl)-imidazol, Smp. 145 – 147° C;
Smp. des Hydrochlorids 250° C (Zersetzung),
4,5-Di-t-butyl-2-(5-methoxy-3,4-methylendioxyphenyl)-imidazol, Smp. 110 – 113° C;
Smp. des Hydrochlorids 210° C (Zersetzung),
4,5-Di-t-butyl-2-(3,4,5-trimethyloxyphenyl)-imidazol, Smp. 140 – 142° C;
Smp. des Hydrochlorids 230° C (Zersetzung),
4,5-Di-t-butyl-2-phenyl-N-methylimidazol, Smp. 115 – 117° C;
Smp. des Hydrochlorids 205° C (Zersetzung).

Das zur Herstellung des letztgenannten N-methylimidazols verwendete Ausgangsthiepinoimidazol kann wie folgt hergestellt werden:

Unter Stickstoff oder Argon wird eine Suspension von 0,44 g Natriumhydrid (55% in Paraffin) in 10 ml Dimethylformamid auf 0° C abgekühlt. Hierauf wird eine Lösung von 2,86 g 2-Phenyl-4,5,7,8-tetrahydro-4,4,8,8-tetramethyl-1-H-thiepino[4,5-d]imidazol gelöst und 10 ml Dimethylsulformamid zugetropft. Man läßt 20 Minuten bei Raumtemperatur ausreagieren. Dann werden 2,1 g Methyljodid in 10 ml Dimethylformamid zugetropft. Man läßt noch 30 Minuten bei Raumtemperatur ausreagieren. Die Reaktionsmischung wird unter Rühren auf Eiswasser gegossen, der Niederschlag abfiltriert und in Wasser gewaschen. Der Filterrückstand wird in Äther gelöst und die Lösung getrocknet und eingeengt. Die Suspension wird mit Petroläther versetzt. Nach der Kristallisation wird filtriert und das Produkt aus n-Heptan umkristallisiert. Das 2-Phenyl-4,5,7,8-tetrahydro-1,4,4,8,8-pentamethyl-1-H-thiepino[4,5]imidazol schmilzt bei 158 – 160° C. Smp. des Hydrochlorids: 240° C (Zersetzung).

Beispiel 3

5,8 g 2-(3,4-Methylendioxyphenyl)-4,5,7,8-tetrahydro-4,4,8,8-tetramethyl-1-H-thiepino[4,5-d]imidazol, 200 ml Tetrahydrofuran und Nickel-Katalysator (erhalten durch Entwässerung von 85 g feuchtem Nickel-Katalysator mit 300 ml Tetrahydrofuran, Aufwirbeln gefolgt durch Sedimentation des Katalysators und Absaugen des Lösungsmittels) werden 24 Stunden bei Rückflußtemperatur erhitzt. Die Reaktionslösung wird wie im Beispiel 1 jedoch unter Verwendung von Tetrahydrofuran an Stelle von Dioxan behandelt. Nach Umkristallisation aus n-Hexan erhält man 3,5 g 4,5-Di-t-butyl-2-(3,4-methylendioxyphenyl)-imidazol, Smp. 175 – 178° C.

In zu Beispiel 1 analoger Weise erhält man aus 3,2 g Base 3,5 g Hydrochlorid, Smp. 200° C (Zersetzung).

Beispiel 4

In üblicher Weise werden Tabletten folgender Zusammensetzung hergestellt:

| | |
|---|---|
| Di-t-butyl-2-(4-fluorphenyl)-imidazol-hydrochlorid | 185,0 mg |
| Milchzucker | 15,0 mg |
| Maisstärke | 37,9 mg |
| Wasserlösliches Polyvinylpyrrolidon | 10,0 mg |
| Magnesiumstearat | 2,5 mg |
| Gesamtgewicht pro Tablette | 250,0 mg |

Beispiel 5

In üblicher Weise werden Gelatinesteckkapseln folgender Zusammensetzung hergestellt:

| | |
|---|---|
| Di-t-butyl-2-(4-fluorphenyl)-imidazol-hydrochlorid | 200,0 mg |
| Wasserlösliches Polyvinylpyrrolidon | 2,0 mg |
| Maisstärke | 43,0 mg |
| Talk | 4,5 mg |
| Magnesiumstearat | 0,5 mg |
| Gesamtgewicht pro Kapsel | 250,0 mg |

Beispiel 6

In üblicher Weise wird eine Injektionslösung folgender Zusammensetzung hergestellt:

| | |
|---|---|
| Di-t-butyl-2-(4-fluorphenyl)-imidazol-hydrochlorid | 115,0 mg |
| Glycerinformal | 2,4 ml |
| Wasser | 4,0 ml |

**Patentansprüche für die Vertragsstaaten: BE, CH, DE, FR, GB, IT, LI, LU, NL, SE**

1. Imidazolderivate der Formel

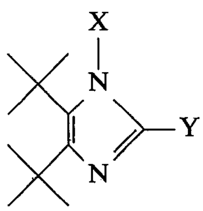

(I)

worin X Wasserstoff oder $C_{1-4}$-n-Alkyl und Y gegebenenfalls durch Methyl oder Fluor substituiertes Thienyl oder eine Gruppe der Formel

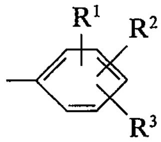

($Y^1$)

ist, worin $R^1$, $R^2$ und $R^3$ Wasserstoff, Methyl, Fluor, Hydroxy, Methoxy oder zwei benachbarte Reste $R^1$, $R^2$ oder $R^3$ zusammen Methylendioxy oder Äthylendioxy sind oder einer der Reste $R^1$, $R^2$ und $R^3$ Mono- oder Di-($C_{1-4}$-n-alkyl)-amino ist und die zwei anderen Wasserstoff sind,
und physiologisch verträgliche Säureadditionssalze davon.

2. Verbindungen nach Anspruch 1, worin X Wasserstoff ist.

3. Verbindungen nach Anspruch 1 oder 2, worin Y eine Gruppe der Formel $Y^1$ ist.

4. Verbindungen nach Anspruch 1, 2 oder 3, worin Y p-Fluorphenyl ist.

5. 4,5-Di-t-butyl-2-(4-fluorphenyl)-imidazol.

6. Die Verbindungen 4,5-Di-t-butyl-2-phenylimidazol, 4,5-Di-t-butyl-2-(4-methoxyphenyl)-imidazol, 4,5-Di-t-butyl-2-(5-methoxy-3,4-methylendioxyphenyl)-imidazol, 4,5-Di-t-butyl-2-(3,4,5-trimethoxyphenyl)-imidazol, 4,5-Di-t-butyl-2-phenyl-N-methylimidazol und Di-t-butyl-2-(3,4-methylendioxyphenyl)-imidazol.

7. Eine Verbindung nach einem der Ansprüche 1–6 als pharmazeutischer Wirkstoff.

8. Eine Verbindung nach einem der Ansprüche 1–6 als die Blutplättchenaggregation hemmender Wirkstoff.

9. Pharmazeutisches Präparat, enthaltend eine Verbindung nach einem der Ansprüche 1–6.

10. Pharmazeutisches Präparat zur Hemmung der Blutplättchenaggregation, enthaltend eine Verbindung nach einem der Ansprüche 1–6.

11. Verfahren zur Herstellung von Verbindungen der Formel I in Anspruch 1 und von physiologisch verträglichen Säureadditionssalzen davon, dadurch gekennzeichnet, daß man ein Thiepinoimidazol der Formel

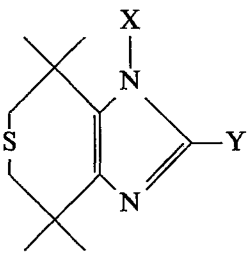

(II)

worin X und Y die obige Bedeutung haben, oder ein Säureadditionssalz davon mit einem Entschwefelungskatalysator behandelt und gewünschtenfalls eine Verbindung der Formel I in ein physiologisch verträgliches Säureadditionssalz überführt.

**Patentansprüche für den Vertragsstaat: AT**

1. Verfahren zur Herstellung von Imidazolderivaten der Formel

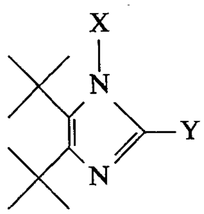

(I)

worin X Wasserstoff oder $C_{1-4}$-n-Alkyl und Y gegebenenfalls durch Methyl oder Fluor substituiertes Thienyl oder eine Gruppe der Formel

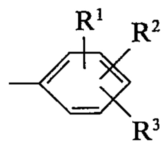

($Y^1$)

ist, worin $R^1$, $R^2$ und $R^3$ Wasserstoff, Methyl, Fluor, Hydroxy, Methoxy oder zwei benachbarte Reste $R^1$, $R^2$ oder $R^3$ zusammen Methylendioxy oder Äthylendioxy sind oder einer der Reste $R^1$, $R^2$ und $R^3$ Mono- oder Di-($C_{1-4}$-n-alkyl)-amino ist und die zwei anderen Wasserstoff sind, dadurch gekennzeichnet, daß man ein Thiepinoimidazol der Formel

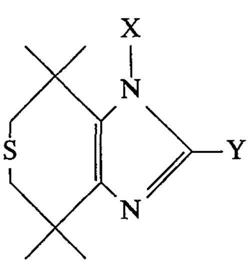

(II)

worin X und Y die obige Bedeutung haben, oder ein Säureadditionssalz davon mit einem Entschwefelungskatalysator behandelt und gewünschtenfalls eine Verbindung der Formel I in ein physiologisch verträgliches Säureadditionssalz überführt.

2. Verfahren nach Anspruch 1, dadurch gekennzeichnet, daß man von einer Verbindung der Formel II, worin X Wasserstoff ist, oder von einem Säureadditionssalz davon ausgeht.

3. Verfahren nach Anspruch 1 oder 2, dadurch gekennzeichnet, daß man von einer Verbindung der Formel II, worin Y eine Gruppe der Formel $Y^1$ ist, oder von einem Säureadditionssalz davon ausgeht.

4. Verfahren nach Anspruch 1, 2 oder 3, dadurch gekennzeichnet, daß man von einer Verbindung der Formel II, worin Y p-Fluorphenyl ist, oder von einem Säureadditionssalz davon ausgeht.

5. Verfahren nach einem der Ansprüche 1–4, dadurch gekennzeichnet, daß man als Ausgangsmaterial 2-(4-Fluorphenyl)-4,5,7,8-tetrahydro-4,4,8,8-tetramethyl-1-H-thiepino[4,5-d]imidazol oder ein Säureadditionssalz davon verwendet.

6. Verfahren nach Anspruch 1, dadurch gekennzeichnet, daß man 4,5-Di-t-butyl-2-phenylimidazol, 4,5-Di-t-butyl-2-(4-methoxyphenyl)-imidazol, 4,5-Di-t-butyl-2-(5-methoxy-3,4-methylendioxyphenyl)-imidazol, 4,5-Di-t-butyl-2-(3,4,5-trimethoxyphenyl)-imidazol, 4,5-Di-t-butyl-2-phenyl-N-methylimidazol oder Di-t-butyl-2-(3,4-methylendioxyphenyl)-imidazol ausgehend von der entsprechenden Verbindung der Formel II herstellt.

**Claims for the Contracting States: BE, CH, DE, FR, GB, IT, LI, LU, NL, SE**

1. Imidazole derivatives of the formula

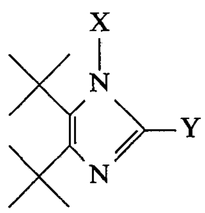

(I)

wherein X is hydrogen or $C_{1-4}$-n-alkyl and Y is thienyl, optionally substituted by methyl or fluorine, or a group of the formula

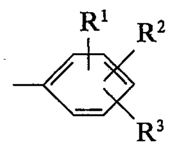

($Y^1$)

wherein $R^1$, $R^2$ and $R^3$ are hydrogen, methyl, fluorine, hydroxy, methoxy or two adjacent residues $R^1$, $R^2$ or $R^3$ are together methylenedioxy or ethylenedioxy or one of the residues $R^1$, $R^2$ and $R^3$ is mono- or di-($C_{1-4}$-n-alkyl)-amino and the other two are hydrogen,
and physiologically compatible acid addition salts thereof.

2. Compounds according to claim 1, wherein X is hydrogen.

3. Compounds according to claim 1 or 2, wherein Y is a group of formula $Y^1$.

4. Compounds accordings to claim 1, 2 or 3, wherein Y is p-fluorophenyl.

5. 4,5-Di-t-butyl-2-(4-fluorophenyl)-imidazole.

6. The compounds 4,5-di-t-butyl-2-phenylimidazole, 4,5-di-t-butyl-2-(4-methoxyphenyl)-imidazole, 4,5-di-t-butyl-2-(5-methoxy-3,4-methylenedioxyphenyl)-imidazole, 4,5-di-t-butyl-2-(3,4,5-trimethoxyphenyl)-imidazole, 4,5-di-t-butyl-2-phenyl-N-methylimidazole and di-t-butyl-2-(3,4-methylenedioxyphenyl)-imidazole.

7. A compound according to any one of claims 1–6 as a pharmaceutically active substance.

8. A compound according to any one of claims 1–6 as an active substance which inhibits blood platelet aggregation.

9. Pharmaceutical preparation containing a compound according to any one of claims 1–6.

10. Pharmaceutical preparation for inhibiting blood platelet aggregation, containing a compound according to any one of claims 1–6.

11. Process for the manufacture of compounds of formula I in claim I and of physiologically compatible acid addition salts thereof, characterized by treating a thiepinoimidazole of the formula

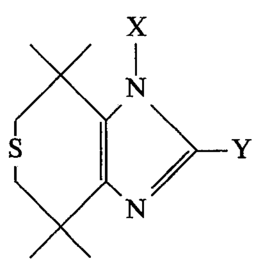

(II)

wherein X and Y have the above significance,
or an acid addition salt thereof with a desulphurizing catalyst and, if desired, converting a compound of formula I into a physiologically compatible acid addition salt.

**Claims for the Contracting State: AT**

1. Process for the manufacture of imidazole derivatives of the formula

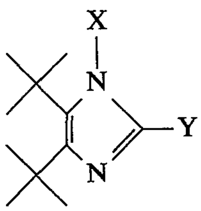

(I)

wherein X is hydrogen or $C_{1-4}$-n-alkyl and Y is thienyl, optionally substituted by methyl or fluorine, or a group of the formula

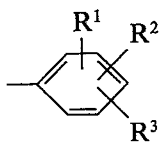

($Y^1$)

wherein $R^1$, $R^2$ and $R^3$ are hydrogen, methyl, fluorine, hydroxy, methoxy or two adjacent residues $R^1$, $R^2$ or $R^3$ are together methylenedioxy or ethylenedioxy or one of the residues $R^1$, $R^2$ and $R^3$ is mono- or di-($C_{1-4}$-n-alkyl)-amino and the other two are hydrogen,
characterized by treating a thiepinoimidazole of the formula

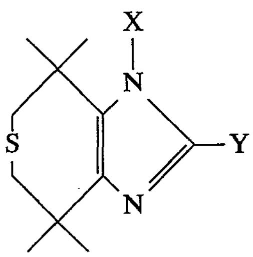

(II)

wherein X and Y have the above significance,
or an acid addition salt thereof with a desulphurizing catalyst and, if desired, converting a compound of formula I into a physiologically compatible acid addition salt.

2. Process according to claim 1, characterized in that a compound of formula II wherein X is hydrogen or an acid addition salt thereof is used.

3. Process according to claim 1 or 2, characterized in that a compound of formula II wherein Y is a group of formula $Y^1$ or an acid addition salt thereof is used.

4. Process according to claim 1, 2 or 3, characterized in that a compound of formula II wherein Y is p-fluorophenyl or an acid addition salt thereof is used.

5. Process according to any one of claims 1—4, characterized in that 2-(4-fluorophenyl)-4,5,7,8-tetrahydro-4,4,8,8-tetramethyl-1-H-thiepino[4,5-d]imidazole or an acid addition salt thereof is used as the starting material.

6. Process according to claim 1, characterized in that 4,5-di-t-butyl-2-phenylimidazole, 4,5-di-t-butyl-2-(4-methoxyphenyl)-imidazole, 4,5-di-t-butyl-2-(5-methoxy-3,4-methylenedioxyphenyl)-imidazole, 4,5-di-t-butyl-2-(3,4,5-trimethoxyphenyl)-imidazole, 4,5-di-t-butyl-2-phenyl-N-methylimidazole or di-t-butyl-2-(3,4-methylenedioxyphenyl)-imidazole is manufactured starting from the corresponding compound of formula II.

**Revendications pour les Etats contractants: BE, CH, DE, FR, GB, IT, LI, LU, NL, SE**

1. Dérivés d'imidazole de formule

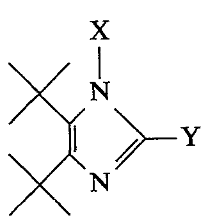

(I)

où X représente un hydrogène ou un n-alcoyle en $C_1$ à $C_4$ et Y est un thiényle éventuellement substitué par un méthyle ou un fluor ou un groupe de formule

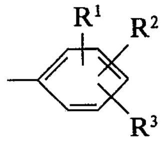

(Y[1])

où $R^1$, $R^2$ et $R^3$ représentent un hydrogène, un méthyle, un fluor, un hydroxy, un méthoxy, ou deux radicacaux $R^1$, $R^2$ ou $R^3$ voisins forment ensemble un méthylènedioxy ou un éthylènedioxy ou l'un des radicaux $R^1$, $R^2$ et $R^3$ est un mono- ou di-(n-alcoyle en $C_1$ à $C_4$)-amino et les deux autres sont des hydrogènes,
et leurs sels d'addition d'acides physiologiquement acceptables.

2. Composés selon la revendication 1, où X est un hydrogène.
3. Composés selon l'une des revendications 1 ou 2, où Y est un groupe de formule $Y^1$.
4. Composés selon l'une des revendications 1, 2 ou 3, où Y est un p-fluorophényle.
5. 4,5-di-t-butyl-2-(4-fluorophényl)-imidazole.
6. Les composés 4,5-di-t-butyl-2-phénylimidazole, 4,5-di-t-butyl-2-(4-méthoxyphényl)-imidazole, 4,5-di-t-butyl-2-(5-méthoxy-3,4-méthylènedioxyphényl)-imidazole, 4,5-di-t-butyl-2-(3,4,5-triméthoxyphényl)-imidazole, 4,5-di-t-butyl-2-phényl-N-méthylimidazole et di-t-butyl-2-(3,4-méthylènedioxyphényl)-imidazole.
7. Composé selon l'une des revendications 1 – 6 comme substance active pharmaceutique.
8. Composé selon l'une des revendications 1 – 6 comme substance active inhibant l'agglutination des plaquettes sanguines.
9. Préparation pharmaceutique contenant un composé selon l'une des revendications 1 – 6.
10. Préparation pharmaceutique pour inhiber l'agglutination des plaquettes sanguines, contenant un composé selonl 'une des revendications 1 – 6.
11. Procédé de préparation de composés de formule I de la revendication 1 et de leurs sels d'addition d'acides physiologiquement acceptables, caractérisés en ce qu'on traite un thiépinoimidazole de formule

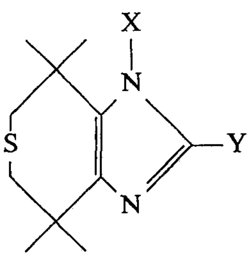

(II)

où X et Y ont la signification donnée ci-dessus,
ou un de ses sels d'addition d'acides, avec un catalyseur de désulfuration et si on le désire en ce qu'on transforme un composé de formule I en un sel d'addition d'acide physiologiquement acceptable.

**Revendications pour l'Etat contractant: AT**

1. Procédé de préparation de dérivés d'imidazole de formule

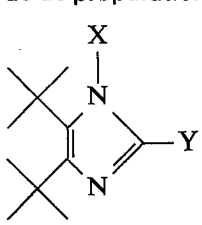

(I)

où X représente un hydrogène ou un n-alcoyle en $C_1$ à $C_4$ et Y un thiényle éventuellement substitué par un méthyle ou un fluor, ou un groupe de formule

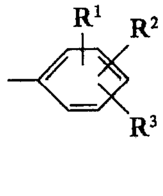

(Y[1])

où $R^1$, $R^2$ et $R^3$ représentent un hydrogène, un méthyle, un fluor, un hydroxy, un méthoxy, ou bien où deux radicaux $R^1$, $R^2$ ou $R^3$ voisins forment ensemble un méthylènedioxy ou un éthylènedioxy ou l'un des radicaux $R^1$, $R^2$ et $R^3$ est un mono- ou un di(n-alcoyle en $C_1$ à $C_4$)-amino et les deux autres sont des hydrogènes,

caractérisé en ce qu'on traite un thiépinoimidazole de formule

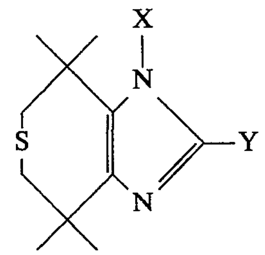

(II)

où X et Y ont la signification donnée ci-dessus,

ou un de ses sels d'addition d'acides, avec un catalyseur de désulfuration, et si on le désire en ce qu'on transforme un composé de formule I en un sel d'addition d'acide physiologiquement acceptable.

2. Procédé selon la revendication 1, caractérisé en ce qu'on part d'un composé de formulue II où X est un hydrogène, ou d'un de ses sels d'addition d'acides.

3. Procédé selon l'une des revendications 1 ou 2, caractérisé en ce qu'on part d'un composé de formule II où Y est un groupe de formule $Y^1$, ou d'un de ses sels d'addition d'acides.

4. Procédé selon l'une des revendications 1, 2 ou 3, caractérisé en ce qu'on part d'un composé de formule II où Y est un p-fluorophényle, ou d'un de ses sels d'addition d'acides.

5. Procédé selon l'une des revendications 1—4, caractérisé en ce qu'on utilise comme produit de départ le 2-(4-fluorophényl)-4,5,7,8-tétrahydro-4,4,8,8-tétraméthyl-1-H-thiépino[4,5-d]imidazole ou un de ses sels d'addition d'acides.

6. Procédé selon la revendication 1, caractérisé en ce qu'on prépare le 4,5-di-t-butyl-2-phénylimidazole, le 4,5-di-t-butyl-2-(4-méthoxyphényl)-imidazole, le 4,5-di-t-butyl-2-(5-méthoxy-3,4-méthylèndioxyphényl)-imidazole, le 4,5-di-t-butyl-2-(3,4,5-triméthoxyphényl)-imidazole, le 4,5-di-t-butyl-2-phényl-N-méthylimidazole ou le di-t-butyl-2-(3,4-méthylènedioxyphényl)-imidazole en partant du composé correspondant de formule II.